# EUROPEAN PATENT SPECIFICATION

(11) **EP 2 157 180 B1**
(45) Date of publication and mention of the grant of the patent: **04.03.2015**
(21) Application number: 08794073.0
(22) Date of filing: 11.07.2008
(51) Int. Cl.: C12Q 1/68, C12N 15/10, C12P 19/34

(54) **METHOD FOR AMPLIFYING SPECIFIC NUCLEIC ACID FRAGMENTS WITH THE AID OF A RECURRENT CHAIN REACTION**
VERFAHREN ZUR VERSTÄRKUNG BESTIMMTER NUKLEINSÄUREFRAGMENTE MITHILFE EINER WIEDERKEHRENDEN KETTENREAKTION
PROCÉDÉ D'AMPLIFICATION DE FRAGMENTS SPÉCIFIQUES D'ACIDES NUCLÉIQUES PAR RÉACTION EN CHAÎNE RÉCURRENTE

(30) Priority: 14.06.2007 RU 2007121892
(43) Date of publication of application: 24.02.2010
(73) Proprietor: Institut Biokhimii I Genetiki Ufimskogo Nauchnogo Tsentra Ran, Ufa 450054 (RU)
(72) Inventor: CHEMERIS, Dmitry Alekseevich, Ufa, 450097 (RU); CHEMERIS, Aleksei Viktorovich, Ufa, 450097 (RU); NIKONOROV, Yury Mikhailovich, Ufa, 450000 (RU); MAGDANOV, Eduard Gavisovich, Ufa, 450077 (RU); MAGAZOVA, Roza Azatovna, Ufa, 490097 (RU); VAKHITOV, Vener Absatarovich, Ufa, 450006 (RU); MIKHAILENKO, Konstantin Ivanovich, Ufa, 450068 (RU)
(74) Representative: Henriksson, Dan Ragnar Mikael
(86) International application number: PCT/RU2008/000461
(87) International publication number: WO 2008/153446

(56) References cited:
- EP-A1- 0 684 315
- WO-A1-97/20948
- US-A1- 2004 063 144
- US-B1- 6 479 244
- ZHANG YUANLI ET AL: "A novel real-time quantitative PCR method using attached universal template probe", NUCLEIC ACIDS RESEARCH, OXFORD UNIVERSITY PRESS, GB, vol. 31, no. 20, 15 October 2003 (2003-10-15), pages E123-E132, XP002498258, ISSN: 1362-4962, DOI: DOI:10.1093/NAR/GNG123
- ZHANG Y. ET AL.: 'A novel real-time quantitative PCR method using attached universal template probe' NUCLEIC ACIDS RESEARCH vol. 31, no. 20, 2003, pages 1 - 8, XP002498258
- MAZARS G-R. ET AL.: 'Direct sequencing by thermal asymmetric PCR' NUCLEIC ACIDS RESEARCH vol. 19, no. 17, 1991, page 4783, XP002532640

## Description

The invention relates to molecular biology and biotechnology and is related to the amplification and analysis of DNA and RNA molecules. It may be used for sequencing DNA, for DNA diagnostics in medicine, veterinary science, in sanitary and epidemiological studies, in the food industry for detecting food products made from genetically modified organisms, for determining raw material quality, for detecting the agents of dangerous infections, including potential bio-terrorist attacks, and in criminalistics for identifying criminals, etc.

The existing methods for sequencing DNA and DNA diagnostics are based primarily on amplification of specific DNA or RNA fragments with the aid of polymerase chain reaction (PCR) and modification thereof. In recent years, the real time PCR is being used more and more, wherein the detection of the target product is carried out directly during amplification with the aid of special DNA thermocyclers equipped with an optical module. One of the important advantages of such an approach is the absence of the necessity of carrying out the step of electrophoretic separation of the reaction products, which presumes opening the test tubes and manipulating the content thereof in air, as a result of which the working zone of the place is contaminated with PCR products - amplicons, which are billions or even trillions of copies, resulting in their being followed in the course of subsequent analyses by the obtainment of falsely-positive results, which were obtained because new reaction mixtures may be initially contaminated in a droplet manner with amplicons produced during preceding positive reactions and circulating in the air. In addition to this, the possibility of not conducting electrophoretic analysis significantly reduces the time for the whole procedure. Another important advantage is the possibility for the exact quantitative determination with the aid of real time PCR of the number of copies of that or other target, for example, the content of the genetically modified ingredients or any other impurities in the raw material or in the food products.

Upon the use of PCR products for sequencing, it is desirable to use single-stranded DNA, which can be achieved in different ways. So, in order to separate amplicon chains, one of which carries a biotin label in the structure of the primer, magnetic particles are used that are coated with streptavidin [Hultman et al., 1989]. Simpler methods of obtaining the single-stranded DNA for sequencing are provided by different variants of asymmetrical DNA amplification with the aid of PCR [Gyllensten, Erlich, 1988; Mazars et al., 1991; Sanchez et al., 2006], providing however only the main accumulation of one of the chains and on the whole reducing the effectiveness of the PCR itself.

In the overwhelming majority of cases, classical PCR is carried out with a pair or primers, which are generally designated as "forward" and "reverse". The sequences of the primers are selected on such a basis so that they would anneal on corresponding DNA fragments against one another, thus providing exponential amplification. Usually the primers are completely homologous to the places of annealing, with the exception of special cases directed either to the discrimination of some or other DNA fragments, which differ by single or multiple replacements of nucleotides, and in that case the unpaired nucleotides may be arranged at the 3'-end of the primer or near it [Gibbs et al., 1989; Huang et al., 1992]. Another reason for the incomplete homology of the primer with the place of annealing is the conducted site-directed mutagenesis and then the unpaired nucleotide on which it is desired that a specific sequence be changed is usually positioned in the middle part of the primer [Hemsley et al., 1989]. Primers with unpaired parts at the 5'-end are usually used in those cases when it is desired to introduce a recognition site of one or another restriction endonuclease for the subsequent use thereof in molecular cloning [Scharf et al., 1986]. An unpaired section at the 5'-end of the primer may also serve as a place for annealing some universal hybridization probe [Zhang et al., 2003].

In addition to the usually used in PCR pair of primers, in some cases with the aim of achieving enhanced specificity of the reaction of an enhanced sensitivity, two pairs of primers are used that are external and internal in respect to one another and such a method of carrying out PCR is called "nested" [Erlich et al., 1991]. Wherein, when such PCR is carried out there are variants with stopping the reaction and adding a second pair of primers that are internal in respect to the first. A simpler and more convenient variant is the use of pairs of primers, which significantly differ in respect to their temperature of annealing. As a result of this at the first step with a high temperature of annealing, amplification takes place only with pairs of external primers, while in the second step upon a corresponding reduction of the temperature of annealing, the second pair of internal primers is included in the amplification [Yourno, 1992].

A method of amplifying is also known, which is called by the authors Linked Linear Amplification or abbreviated LLA [Reyes et al., 2001; Killen et al., 2003], at the base of which is essentially the same PCR, but multiple pairs of nested primers (up to 7-9 pairs) are used that are **characterized in that** near the 3'-end of each of them instead of a corresponding nitrogenous base there is an element that is not replicable, blocking the structure of a new DNA chain based thereon in the following cycle. So, new places for annealing for these primers do not arise in the course of amplification, which does not make it possible for exponential amplification to take place. Nevertheless, due to the large number of pairs of primers, the sum reproduction of the central part of the required DNA fragment may be compared with the same in PCR. It was noted by other authors that if only one primer is made from each pair with an element that is not replicable, then the multiplication coefficient increases [Behlke et al., 2006], which, by the way, is not surprising. The increased specificity of these reactions is underlined by the authors of these published materials, but the specificity was reached at a high price, since an increase of the number of pairs of primers makes the reaction significantly more expensive and in the case of using at least a part of the primers of full worth (without an element that is not replicable) makes it in fact more unpredictable.

So, the LLA method together with the nested PCR to a certain degree serves as prototype of the new reaction that we have proposed, wherein PCR is the base thereof, but due to the fact that there are relatively sharp distinctions, consisting mainly in the character of accumulating target products, exceeding PCR in that respect by several orders, we decided to name this reaction in another way - Recurrent Chain Reaction or abbreviated RCR. Actually, the majority of existing reactions of amplification of nucleic acids with a more than linear accumulation of target products are essentially recurrent or recursive. However, in addition to the existing mathematical term "recurrent," the main meaning of this word characterizes events taking place as "repeating periodically, from event to event," which to a certain degree corresponds to the character of annealing primers in the RCR. So, in order to eliminate the long name of this reaction as the type "PCR with an increasing in each cycle coefficient of amplifying DNA molecules (or with an increasing in each cycle number of places for annealing primers within an amplicon)" and to distinguish this new wording from the usual PCR we took the decision to name it in a rather standard and short manner - RCR. Strictly speaking, the name PCR is not sufficiently capacious, since only the used enzyme - polymerase, is mentioned therein and there is not any information at all concerning DNA as another, not less important, component of the reaction mixture. The name of the reaction LLA, for example, is even less informative. Therefore, it is our opinion that the introduced designation RCR may have the right to exist and in the case of broad use of this reaction, it seems to us that after some time upon the mention of this abbreviation it will immediately be clear what consideration is being given to and there will be no need to decipher what this PCR is.

The object of the invention consists in the possibility of carrying out amplification of specific DNA (or RNA with the participation of reverse transcriptase or DNA polymerase, having such activity) fragments with increasing with each cycle coefficient of replication, due to which accumulation of target amplicons takes place significantly faster. On the one hand this reduces the reaction time and on the other increases the sensitivity thereof, making it possible to confidently detect single molecules of nucleic acids in only 20-25 cycles without using the nested PCR or without the second step of PCR with an aliquot part from the first reaction with new ingredients. In addition to this in one of the variants of this reaction there is mainly accumulation of one of the DNA chains, which may be used upon enzymatic sequencing DNA with dideoxy terminators.

The essence of the invention consists in that instead of the primers that are usual for a standard PCR, which as a rule are completely homologous in respect to the places of annealing, primers are used in the RCR as forward and reverse or only as one of them, which primers are in tandem repeating sequences of a single (main) primer with the positioning of repeats in them according to the "head to tail" type and consisting of two or a larger number of such elements. If for a usual primer the whole sequence of nucleotides therein is shown in the form of the letter "a," then the duplicate primer will be designated as "aa," the triplicate - "aaa". In the form of a nucleotide record they may be presented, for example, as 5'-gttcgtcgagtctcgtgtta-3' (usual, here - single or main), 5'-gttcgtcgagtctcgtgttagttcatcaafltctcgtgtta-3' (doublet), 5'-gttcgtcgagtctcgtgttagttcgtcgagtctcgtgttagttcgtcgagtctcgtgtta-3' (triplet), where the two underlined (differently) parts are complete copies of the not underlined. In addition to the fact that with each cycle elongation of the amplicon also takes place by the length of a single primer (upon use of a doublet or by the length of a doublet upon use of a triplet) and there is an increase of the annealing places for them in the structure of the amplicon, the use of a thermostable DNA polymerase of the Vent type with strand displacement activity results in the occurrence of a double-stranded amplicon in each cycle, and also amplified single-stranded DNA chains before annealed primers, displaced by such polymerase, wherein the number of such amplicons in the form of single-stranded DNA chains grows with each cycle.

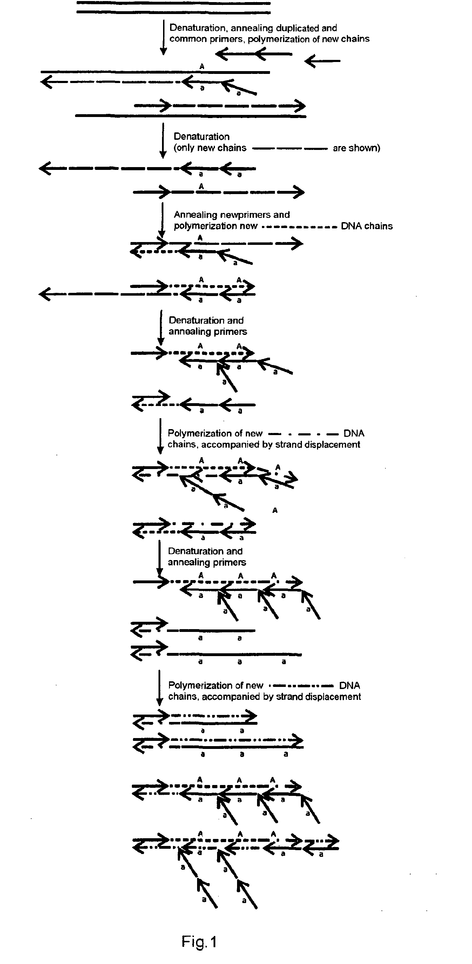

As is evident from Fig. 1, upon annealing such primers that consist of tandem repeats of a singular primer on an initial nucleotide sequence carrying only one annealing place, which may be designated as "A," an unpaired section is formed as one of two "a"s. Wherein, if the primer is annealed at its main part, positioned closer to the 3'-end, already after a cycle, such sections for annealing primers in the structure of the amplicon will be two "AA"s, and theoretically, for example, two doublet primers "aa" and "aa" may anneal on them. Wherein, a thermostable DNA polymerase with strand displacement activity as a result of elongation of the primer annealed at the external place of annealing displaced the DNA chain that is encountered in its path and was amplified with the aid of a primer annealed at the internal place, which could somewhat remind the nested PCR with simultaneous annealing of the internal and external primers in the case of using DNA polymerase with a shifting DNA activity chain. However, in the nested PCR, an increase in the number of places of annealing the primer does not take place and each of them (external and internal) "works" independently of the other. With each cycle, elongation of the amplicon takes place in the RCR due to an increase of the number of annealing places, as a result of which an ever greater number of doublet primers "aa" may anneal on growing AA-, AAA-, AAAA- etc. parts. An increase of the places of annealing will also take place in a similar manner for the other chain provided that at least a doublet is used as the second primer.

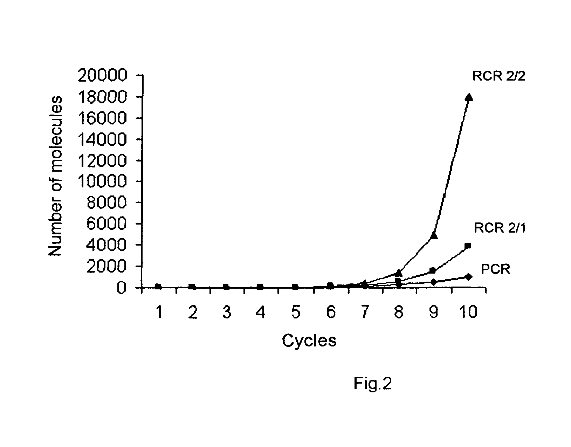

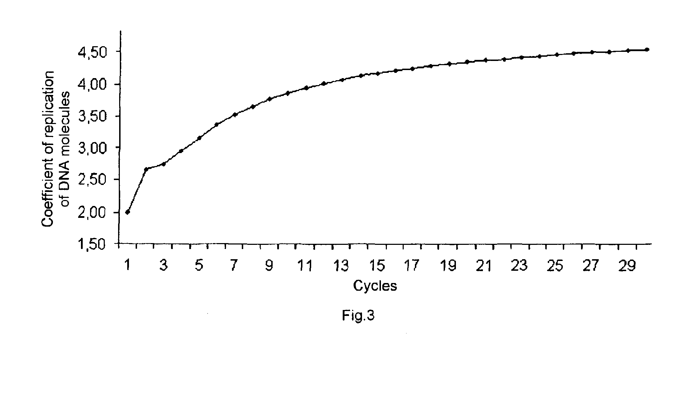

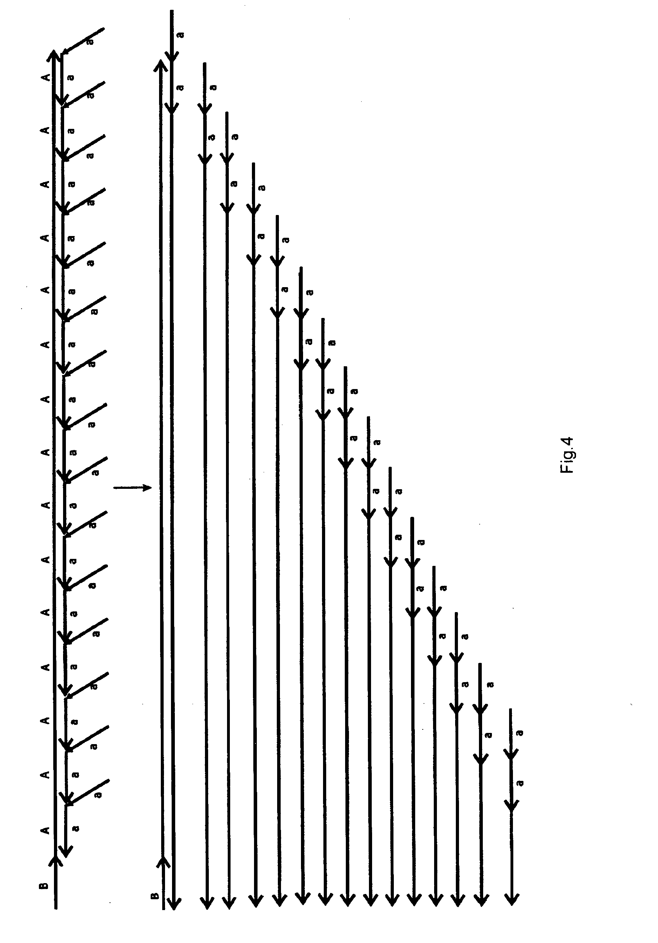

An RCR using one singular (common) and one doublet primer is designated by us as RCR 2/1. An RCR using doublet primers as the forward and reverse is designated in a similar manner as RCR 2/2. An increase of the accumulation of amplicons in these reactions significantly exceeds the same in a common PCR, which is evident from Fig. 2. So, for a PCR, the maximum theoretically possible coefficient of replication is equal to two provided there is 100% effectiveness of this reaction. In the RCR the coefficient of replication of DNA molecules increases with each cycle (Fig. 3) and at the 20^{th} cycle for RCR 2/2 it is theoretically on the average 4.35, wherein in the case of amplicons appearing for example in the first cycles it will be 8.5 and 8 at that moment, also theoretically. In view of the aforesaid, it follows that after completion of the tenth cycle, for example, it may be considered that amplification of the initial DNA sequences in the PCR, taking into account the heterogeneous by length amplicons formed upon annealing primers on the initial DNA chains and these chains themselves, theoretically occurs 2¹⁰ times or 1024 times. For the same number of cycles, the initial number of specific fragments of DNA in RCR 2/1 and RCR 2/2 theoretically increases by about 4000 and 18000 times respectively, and with each cycle this difference will increase avalanche-like. At the 20^{th} cycle, the difference in the degree of amplification between PCR and RCR 2/2 reaches 5 million times, and at the 30^{th} cycle it exceeds 8 orders. For RCR 2/1, with each cycle the number of single-stranded amplicons also increases, which already after 20 cycles may theoretically be about 1 billion chains, which are immediately suitable for annealing sequencing primers on them (Fig. 4). Wherein, in RCR 2/2 single-stranded amplicons are formed in each cycle, but in this variant of the reaction, such single-stranded amplicons correspond to them that are generated as progenies of the second complementary chain of the initial DNA and are capable of forming with similar progenies the first two-chain structure and thus of competing for an annealing place with a sequencing primer.

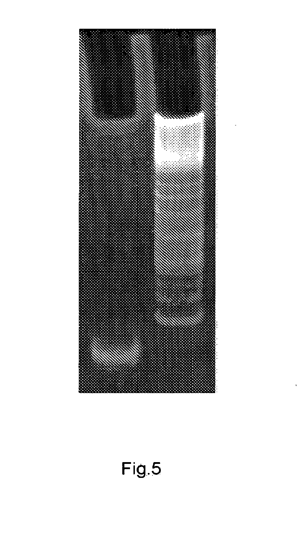

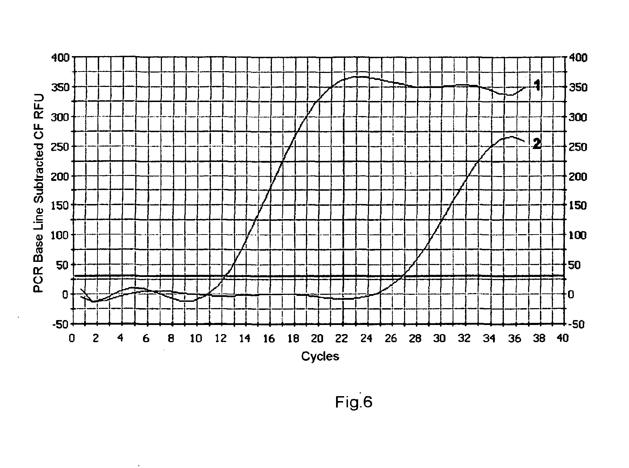

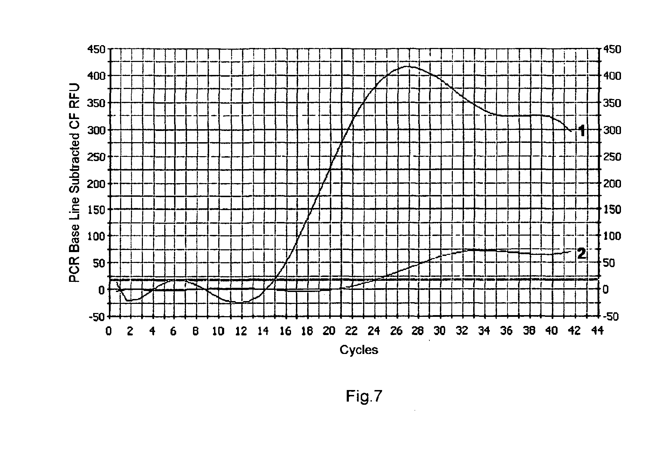

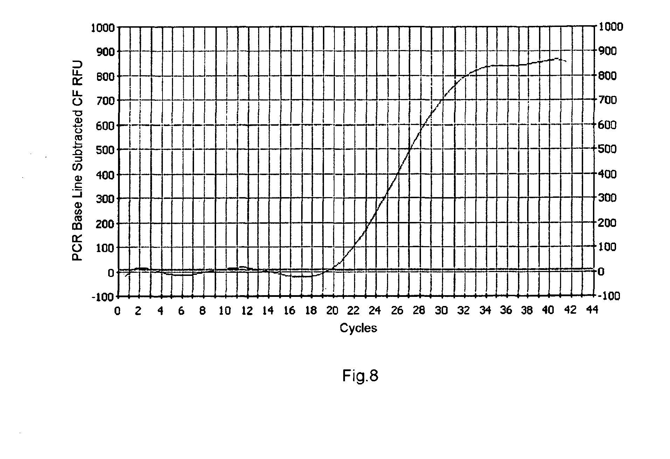

In reality, the effectiveness of the replication of the DNA molecules even in the initial RCR cycles will significantly differ from the theoretically calculated in view of the fact that annealing the primers at repeating annealing places takes place recurrently and not always in an ideal manner, as is shown in the diagram in Fig. 1. A more probable event will be the annealing of a doublet primer with the immediate occupation of two annealing places (when such occur), thus reducing the number of displaced chains. Annealing a primer by its second 5'-end part is also possible, this resulting in that thus the annealed primer will not elongated itself and will not cause displacement of the DNA chain, provided that such is structured from a "correctly" annealed primer by its main part, which in respect to the first is internal. But due to the recurrency of annealing the primers in the form of occurring from time to time "correct" annealing thereof by its main singular part positioned closer to the 3'-end, such events undoubtedly take place, which also follows from Fig. 5 in which the electrophoretic separation of RCR products is shown. What is different from amplification with the aid of PCR with common primers (track 1) is that an increase in the length of amplicons is observed in track 2, which shows that the planned "correct" annealing of doublet primers is taking place among other events. It is evident from Fig. 5 that it is virtually impossible because of the multiple bands to carry out an analysis of RCR products at the final point with the aid of gel electrophoresis. However, in real time RCR detection of an increase of the fluorescence of a specific dye of the SYBR Green I type or by detection of the transfer of fluorescent resonance energy from a dye donor to a dye acceptor, which are included respectively in the structure of forward and reverse primers, provided that the latter are annealed at the target at a distance provided by such transfer, is quite possible. The curves of an increase of fluorescence of an intercalating dye SYBR Green I are shown in Fig. 6 for PCR with common primers and for RCR with doublet primers, from which a significantly earlier rise of the curves for RCR is evident, wherein the initial number of DNA targets was the same and was about 20 copies for both reactions. The curves of an increase of fluorescence are shown in Fig. 7 for PCR and RCR with a transfer of the fluorescent resonance energy. However, in view of the specificities of the device that is tuned to carry out normalization of the obtained meanings, arranging it in definite, presently existing, and mainly developed for PCR frames, the increase of fluorescence in RCR does not seem to be very steep, taking into account that the increase of fluorescence in PCR on the background of meanings for RCR seems to be very insignificant. However, if the obtained meanings for RCR are removed and the device is made to recalculate the increase of fluorescence only for PCR, it turns out to be fully acceptable and even steep (Fig. 8). If the straightened increase of fluorescence for PCR is related by extrapolation to such in that experiment for RCR, then the curve of an increase of fluorescence for the latter will go virtually straight up, which in principle corresponds to the character of accumulation of the chains of amplicons for these reactions that are presented in Fig. 2.

The proposed method of amplification with the aid of a recurrent chain reaction of specific DNA or RNA fragments together with their detection within the real time mode and production of single-stranded amplicons is illustrated by the following examples.

### Brief description of the drawings

Fig. 1 is a schema of RCR 2/1 - Recurrent Chain Reaction 2/1, wherein one of the primers is a doublet, a second - a singular, which results in an asymmetrical accumulation of DNA amplification chains.
Fig. 2 shows the character of an increase of the number of amplicons for two variants of a recurrent chain reaction (RCR 2/1 and RCR 2/2) in comparison with polymerase chain reaction (PCR).
Fig. 3 shows an increase of the coefficient of replication of DNA molecules in RCR 2/2, increasing with each subsequent cycle.
Fig. 4 shows the character of copying with the aid of DNA polymerase with chain-mixing activity, formed with the use of singular (forward) and doublet (reverse) primers at the 20^{th} cycle of the amplicon. In accordance with the total amount of formed chains in the form of one double-stranded product and 14 single-stranded products, the coefficient may be considered to be equal to 8.
Fig. 5 shows an image of an electrophoretic analysis of the PCR and RCR products in a 10% polyacrylamide gel, amplified with the aid of thermally stable Vent exo-DNA polymerase during 25 cycles, where the track with use of common primers (PCR) is designated by the number 1, the track, where one of the primers is the doublet (RCR 2/1), by the number 2.
Fig. 6 shows the growth of fluorescence curves an intercalating dye SYBR Green I in RCR and in PCR. The sample 1 designates a curve that is characteristic for RCR 2/1, where one of the primers is doublet, while the sample 2 designates a curve that is characteristic for PCR with the common primers.
Fig. 7 shows the growth of fluorescence curves after FRET-effect (fluorescent resonance energy transfer) in RCR 2/2 and in PCR, where the sample 1 designates a curve that is characteristic for RCR 2/2, where both of the primers are doublet, while the sample 2 designates a curve that is characteristic for PCR with the common primers.
Fig. 8 shows the growth of a fluorescent curve upon carrying out PCR in the same experiment as on Fig. 7 after removal from consideration the meanings for RCR 2/2, due to which as a result of normalization by the device (here DNA cycler model iCycler iQ) of the presented data, the curve (2) of the increase of fluorescence acquires a standard character, and not that which is shown in Fig. 7, showing that the curve 1 in Fig. 7, if extrapolation is used, should rise upwards significantly steeper.

### Example 1

### Carrying out RCR 2/2 and RCR 2/1

RCR was carried out in 25 µl of a reaction mixture containing a buffer (40 mM of Tris-HCl pH8, 0.25 mM MgCl₂, 25 mM KCl); 1 unit of activity Vent exo⁻ DNA polymerase; 0.5 pmol of each of two doublet primers, each marked with its fluorochrome (with a donor and acceptor or a dye and quencher) and a corresponding amount of distilled water. Instead of the primers labeled with fluorochromes, unlabeled were also used. Wherein, a change of the fluorescence was controlled with the aid of an intercalating dye SYBR Green I. RCR was carried out in the DNA thermocycler of the model iCycler iQ (Bio-Rad Laboratories) under the following conditions: denaturation of the double-stranded DNA in the first cycle was carried out at 95°C during 30 sec, then the primers were annealed (55°C, 10 sec), elongated (72°C, 20 sec) with detection of the fluorescence at the end of that step, denaturation of the target product, 20 sec, the number of cycles in different experiments varied from 20 to 30.

The RCR 2/1 differed from RCR 2/2 only by the use of one doublet primer, while the second primer of the pair was singular, which provided for the primary accumulation of one of the DNA chains.

### Example 3

### Electrophoretic analysis of RCR products

Electrophoretic separation of the RCR products was carried out in an 8% polyacrylamide gel in a tris-acetate buffer pH 7.8 in undenaturating conditions at a voltage gradient of 4 V per cm length of the gel in a vertical type device during 4 hours. After completion of electrophoresis, the gel after staining with ethidium bromide is photographed in a photo-documention system Gel Camera System (UVP, Inc.).

### Literature

Behlke M.A., Walder J.A., Manthey J.A. Polynomial amplification of nucleic acids // US Patent No 7,112,406 B2. Sep. 26, 2006.
Erlich H.A., Gelfand D., Sninsky J.J. Recent advances in the polymerase chain reaction // Science. 1991. V.252. P.1643-1651.
Gibbs R.A., Nguyen P.N., Caskey C.T. Detection of single DNA base differences by competitive oligonucleotide printing // Nucleic Acids Res. 1989. V.17. P.2437-2448.
Gyllensten U.B., Erlich H.A. Generation of single-stranded DNA by the polymerase chain reaction and its application to direct sequencing of the HLA-DQA locus // Proc. Natl. Acad. Sci. USA. 1998. V.95. P.7652-7656.
Hemsley A., Arnheim N., Toney M.D., Cortopassi G., Galas D.J. A simple method for site-directed mutagenesis using the polymerase chain reaction // Nucleic Acids Res. 1989. V.17. P.6545-6551.
Huang M.M., Arnheim N., Goodman M.F. Extension of base mispairs by Taq DNA polymerase: implications for single nucleotide discrimination in PCR // Nucleic Acids Res. 1992. V.20. P.4567-4573.
Hultman T., Stahl S., Homes E., Uhlen M. Direct solid phase sequencing of genomic and plasmid DNA using magnetic beads as solid support // Nucl. Acids Res. 1989. V.17. P.4937-4946.
Killeen A.A., Breneman J.W., 3rd, Carillo A.R., Ugozzoli L.A.., Lowery J.D., Liu J., Hixson C.S. Linked linear amplification for simultaneous analysis of the two most common hemochromatosis mutations // Clin. Chem. 2003. V.49. P. 1050-1057.
Mazars G-R., Moyret C., Jeanteur P., Theillet C-G. Direct sequencing by thermal asymmetric PCR // Nucl. Acids Res. 1991. V.19. P.4783.
Reyes A.A., Ugozzoli L.A., Lowery J.D., Breneman J.W., 3rd, Hixson C.S., Press R.D., Wallace R.B. Linked linear amplification: a new method for the amplification of DNA // Clin. Chem. 2001. V.47. P.31-40.
Sanchez J.A., Abramowitz J.D., Salk J.J., Reis A.H., Rice J.E., Pierce K.E., Wangh L.J. Two-temperature LATE-PCR endpoint genotyping // BMC Biotechnol. 2006. V.6. #.44.
Scharf S.J., Horn G.T., Erlich H.A. Direct cloning and sequence analysis of enzymatically amplified genomic sequences // Science. 1986. V.233. P.1076-1078.
Yourno J. A method for nested PCR with single closed reaction tubes // PCR Methods Appl. 1992. V.2. P.60-65.
Zhang Y., Zhang D., Li W., Chen J., Peng Y., Cao W. A novel real-time quantitative PCR method using attached universal template probe // Nucleic Acids Res. 2003. V.31. e123.

## Claims

1. A method for amplifying specific nucleic acid fragments, **characterized in that** due to the use of a thermally stable DNA polymerase having strand displacement activity and of forward and reverse primers, wherein the forward and reverse primers or only one of them are tandem repeating sequences of a single main primer with the positioning of repeats in them according to the "head to tail" type, an increase of the number of annealing places of primers takes place, resulting in a constant increase of the coefficient of replication of DNA molecules, in the course of amplification, and in an accelerated accumulation of amplicons.

2. The method according to claim 1, **characterized in that** the structure of the forward and/or reverse primers includes three or more repeats of the main primer.

3. The method according to claim 1 or claim 2, **characterized in that** the structure of the forward and/or reverse primers, in addition to repeats of the main primers thereof, includes other nucleotide sequences or other molecules.

4. The method according to claim 1 or claim 2, **characterized in that** detection of accumulation of amplicons is carried out according to an increase of fluorescence in a thermocycler with an optical module.

5. The method according to claim 3, **characterized in that** detection of accumulation of amplicons is carried out according to an increase of fluorescence in a thermocycler with an optical module.

6. The method according to claim 1 or claim 2, **characterized in that** one of the pair of primers (forward or reverse) consists of a singular main sequence, while the other is a repeat primer providing an asymmetrical course of the reaction and the appearance of amplicons in the form of single-stranded DNA fragments that are suitable for sequencing thereof by an enzymatic method.

7. The method according to claim 3, **characterized in that** one of the pair of primers (forward or reverse) consists of a singular main sequence, while the other is a repeat primer providing an asymmetrical course of the reaction and the appearance of amplicons in the form of single-stranded DNA fragments that are suitable for sequencing thereof by an enzymatic method.

## Patentansprüche

1. Verfahren zum Amplifizieren bestimmter Nukleinsäurefragmente, **dadurch gekennzeichnet, dass** wegen der Verwendung einer thermostabilen DNA-Polymerase mit Strangverdrängungsaktivität sowie von Vorwärts- und Rückwärts-Primern, wobei es sich bei den Vorwärts- und Rückwärts-Primern oder nur einem von ihnen um Tandem-Wiederholungssequenzen eines einzelnen Hauptprimers handelt, wobei die Wiederholungen darin gemäß dem "Head-to-Tail"-Typ positioniert sind, die Anzahl der Annealing-Orte der Primer steigt, was zu einem konstanten Anstieg des Replikationskoeffizienten von DNA-Molekülen im Verlauf der Amplifikation sowie zu einer beschleunigten Anreicherung von Amplifikaten führt.

2. Verfahren nach Anspruch 1, **dadurch gekennzeichnet, dass** die Struktur der Vorwärts- und/oder Rückwärts-Primer drei oder mehr Wiederholungen des Hauptprimers enthält.

3. Verfahren nach Anspruch 1 oder Anspruch 2, **dadurch gekennzeichnet, dass** die Struktur der Vorwärts- und/oder Rückwärts-Primer neben Wiederholungen der Hauptprimer davon andere Nukleotidsequenzen oder andere Moleküle enthält.

4. Verfahren nach Anspruch 1 oder Anspruch 2, **dadurch gekennzeichnet, dass** der Nachweis der Anreicherung von Amplifikaten entsprechend einem Fluoreszenzanstieg in einem Thermocycler mit einem optischen Modul erfolgt.

5. Verfahren nach Anspruch 3, **dadurch gekennzeichnet, dass** der Nachweis der Anreicherung von Amplifikaten entsprechend einem Fluoreszenzanstieg in einem Thermocycler mit einem optischen Modul erfolgt.

6. Verfahren nach Anspruch 1 oder Anspruch 2, **dadurch gekennzeichnet, dass** ein Primer des Primer-Paars (Vorwärts- oder Rückwärts-Primer) aus einer einmaligen Hauptsequenz besteht, während es sich bei dem anderen um einen Wiederholungs-Primer handelt, was einen asymmetrischen Verlauf der Reaktion und das Auftreten von Amplifikaten in Form von Einzelstrang-DNA-Fragmenten, die sich zur Sequenzierung davon mit einem enzymatischen Verfahren eignen, liefert.

7. Verfahren nach Anspruch 3, **dadurch gekennzeichnet, dass** ein Primer des Primer-Paars (Vorwärts- oder Rückwärts-Primer) aus einer einmaligen Hauptsequenz besteht, während es sich bei dem anderen um einen Wiederholungs-Primer handelt, was einen asymmetrischen Verlauf der Reaktion und das Auftreten von Amplifikaten in Form von Einzelstrang-DNA-Fragmenten, die sich zur Sequenzierung davon mit einem enzymatischen Verfahren eignen, liefert.

## Revendications

1. Procédé d'amplification de fragments d'acide nucléique spécifique, **caractérisé en ce que**, en raison de l'utilisation d'une ADN polymérase thermiquement stable ayant une activité de déplacement devra et d'amorce sent et antisens, les amorces et antisens ou une seule d'entre elle étant des séquences de répétitions en tandem d'une amorce principale unique avec le positionnement de répétitions dans celle-ci conformément au type « tête-à-queue », une augmentation du nombre d'emplacements d'hybridation d'amorce survient, conduisant à une augmentation constante du coefficient de réplication de molécules d'ADN, au cours de l'amplification, et à une accumulation accélérée d'amplicons.

2. Procédé selon la revendication 1, **caractérisé en ce que** la structure des amorces sens et/ou antisens comprend trois répétitions ou plus de l'amorce principale.

3. Procédé selon la revendication 1 ou la revendication 2, **caractérisé en ce que** la structure des amorces sens et/ou antisens, en plus de répétitions des amorces principales de celle-ci, comprend d'autres séquences nucléotidiques ou d'autres molécules.

4. Procédé selon la revendication 1 ou la revendication 2, **caractérisé en ce que** la détection de l'accumulation d'amplicons est conduite en fonction d'une augmentation de la fluorescence dans un thermocycleur avec un module optique.

5. Procédé selon la revendication 3, **caractérisé en ce que** la détection de l'accumulation d'amplicons est conduite en fonction d'une augmentation de fluorescence dans un thermocycleur avec un module optique.

6. Procédé selon la revendication 1 ou la revendication 2, **caractérisé en ce que** l'une de la paire d'amorces (sens ou antisens) est constituée d'une séquence principale unique, tandis que l'autre est une amorce de répétition produisant un déroulement asymétrique de la réaction et l'apparition d'amplicons sous la forme de fragments d'ADN monocaténaires qui sont adaptés pour le séquençage de ceux-ci par un procédé enzymatique.

7. Procédé selon la revendication 3, **caractérisé en ce que** l'une de la paire d'amorces (sens ou antisens) est constituée d'une séquence principale unique, tandis que l'autre est une amorce de répétition produisant un déroulement asymétrique de la réaction et l'apparition d'amplicons sous la forme de fragments d'ADN monocaténaires qui sont adaptés pour le séquençage de ceux-ci par un procédé enzymatique.
